# EUROPÄISCHE PATENTSCHRIFT

(11) **EP 2 263 584 B1**
(45) Veröffentlichungstag und Bekanntmachung des Hinweises auf die Patenterteilung: **05.09.2012**
(21) Anmeldenummer: 10010953.7
(22) Anmeldetag: 07.03.2003
(51) Int. Cl.: A61B 17/72

(54) **Marknagel mit Verriegelungsschraube**
Intramedullary nail with locking screw
Clou intramédullaire avec vis de blocage

(43) Veröffentlichungstag der Anmeldung: 22.12.2010
(62) Teilanmeldung aus: 03704149.8
(73) Patentinhaber: Synthes GmbH, 4436 Oberdorf (CH)
(72) Erfinder: Senn, Peter, 4437 Waldenburg (CH); Schlienger, André, 4142 Münchenstein (CH); Buettler, Markus, 4702 Oensingen (CH)
(74) Vertreter: Lusuardi, Werther

(56) Entgegenhaltungen:
- EP-A- 1 214 914
- EP-A1- 1 281 365
- WO-A-03/015649
- US-A- 4 657 001
- US-A- 4 754 749
- US-A- 6 053 918

## Beschreibung

Die Erfindung bezieht sich auf einen Marknagel und eine Verriegelungsschraube gemäss dem Oberbegriff des Patentanspruchs 1.

Die Verriegelung von Marknägeln gehört zum Stand der Technik. Die Einführung der Verriegelungsschrauben in die Querbohrungen des Marknagels erfolgt entweder mit Hilfe eines bildgebenden Verfahrens (Röntgenkontrolle) oder einer mehr oder weniger komplizierten Zielvorrichtung. In beiden Fällen ist eine gewisse Zielungenauigkeit nicht zu vermeiden, d.h. die Schraubenspitze lässt sich nicht exakt koaxial zur Mittelachse der Querbohrung ausrichten, sondern weicht davon um einen gewissen Betrag ab. Damit die Verriegelungsschraube trotz dieses Zielfehlers in die Querbohrung mündet und durch diese hindurchgebracht werden kann, wird der Aussendurchmesser der Schraube relativ zum Durchmesser der Querbohrung unterdimensioniert. Bleibt die Zielungenauigkeit im Rahmen dieser Unterdimensionierung, so kann die Verriegelungsschraube trotz des Zielfehlers problemlos durch die Querbohrung geführt werden. Allerdings weist nun die Verriegelungsschraube - wegen der Unterdimensionierung - ein gewisses Spiel auf relativ zur Querbohrung.

Dieses Spiel definiert, um welchen Betrag sich die Knochenhauptfragmente, welche mittels Verriegelungsschrauben im entsprechenden Verriegelungsloch fixiert werden, relativ zum Nagel und somit aufgrund der Starrheit des Nagels auch relativ zu anderen mit demselben Nagel befestigten Knochenhauptfragmente bewegen können. Zusammen mit der Flexibilität des Materials und der Gesamtvorrichtung kann dies kumuliert eine Grösse annehmen, welche eine erfolgreiche Heilung verhindert, oder massgeblich verzögert. Um die Anwendbarkeit der Verriegelung für den Chirurgen zu garantieren, ist dieses Spiel zwar unumgänglich, doch ist es klinisch bei gewissen Indikationen (z.B. im Falle von metaphysären Fragmenten) unerwünscht.

Selbst Nägel mit vollem Querschnitt, welche im Verriegelungsloch ein Innengewinde aufweisen können, ist nicht spielfrei. Das Innengewinde verhindert lediglich, dass der Nagel sich axial auf der Verriegelungsschraube bewegen kann.

Hier will die Erfindung Abhilfe schaffen. Der Erfindung liegt die Aufgabe zugrunde, eine Verriegelungsschraube zu schaffen, mit der das vorhandene Spiel zwischen ihr und dem Marknagel eliminiert und die Verriegelungsschraube in der Querbohrung des Marknagels verkeilt werden kann.

Der Oberbegriff des Anspruchs 1 basiert auf der Offenbarung des Patents US-A-605 3 918. Es ist aus dem Patent US-A-4 657 001 bekannt, mit Nuten veßehene Femurplatten und Schenkelhalsschrauben mittels Verkeilungselemente gegeneinander zu immobilisieren.

Die Erfindung löst die gestellte Aufgabe mit einer Verriegelungsschraube, welche die Merkmale des Anspruchs 1 aufweist.

Die durch die Erfindung erreichten Vorteile sind im wesentlichen darin zu sehen, dass dank der erfindungsgemässen Verriegelungsschraube das Spiel zwischen Marknagel und Verriegelungsschraube eliminiert werden kann. Die Erfindung erlaubt aber auch die Schraube in einer ersten Phase mit Spiel einzubringen und dieses erst nachträglich zu eliminieren. Ein weiterer Vorteil besteht darin, dass trotz der Aufhebung des Spiels die Rotierbarkeit der Verriegelungsschraube nicht blockiert wird. Die Vorteile der Beibehaltung der Rotierbarkeit sind vielfältig:
- die Verriegelungsschraube kann auch im spielreduzierten Zustand ein- oder ausgedreht werden (axiale Freiheit); und
- im Falle eines Versagens der Verriegelungsschraube ist es möglich mit gängigen Techniken die Verriegelungsschraube zu extrahieren (durch Ausschlagen oder durch Herausziehen).

Bei einer besonderen Ausführungsform der Erfindung weist der Schraubenschaft eine in Richtung des Durchgangs verlaufende, erste Nut auf, welche vorzugsweise einen teilkreisförmigen oder polygonalen Querschnitt aufweist. Diese Ausführung ist platzsparend, benötigt eine geringere Einpresskraft und garantiert eine gute Führung des Verkeilungselementes.

Bei einer weiteren Ausführungsform weist der Schraubenschaft eine im wesentlichen parallel zur ersten Nut angeordnete, radial um den Rotationswinkel alpha versetzte, zweite Nut auf, welche tiefer ist als die erste Nut. Der Durchgang im Schraubenkopf ist dabei als Langloch ausgebildet ist, in den beide Nuten münden._Zwischen den beiden Nuten ist eine von der ersten Nut zur zweiten Nut ansteigende Rampe angebracht. Der Rotationswinkel alpha kann einen Wert von 40° bis 120°, vorzugsweise von 60° bis 90° aufweisen.

Bei dieser Ausführungsform besteht der Vorteil darin, dass die Verkeilung schneller stattfindet und auch wieder schneller gelöst werden kann. Die Verkeilungskraft wird wesentlich erhöht bei (vergleichbar) geringerem Kraftaufwand, da die Rampe eine Art Übersetzung darstellt. Da die Verkeilungskraft über ein Drehmoment aufgebracht wird benötigt der Anwender nur einen relativ geringen Kraftaufwand.

Bei einer weiteren Ausführungsform verjüngt sich die Nut mit zunehmendem Abstand vom Schraubenkopf im Querschnitt, vorzugsweise konisch. Dadurch findet die Verkeilung oder Spielverminderung einerseits schneller statt, anderseits kann sie aber auch wieder schneller gelöst werden.

Bei einer weiteren Ausführungsform weist die Nut einen Winkel zur Längsachse der Verriegelungsschraube auf, der vorzugsweise kleiner als 5° ist.

Die Nut kann eine Tiefe aufweisen, welche zwischen 1 % und 50%, vorzugsweise zwischen 2 % bis 20 % des Durchmessers des Schraubenschaftes entspricht.

Zweckmässigerweise wird die erfindungsgemässe Verriegelungsschraube in die Querbohrung eines intramedullären Marknagels eingeführt ist, bei welchem der Durchmesser der Querbohrung grösser ist als der Aussendurchmesser ihres Aussengewindes. Bei Einführung eines longitudinales Verkeilungselement in den Durchgang Verriegelungsschraube erfolgt zwischen dem Schraubenschaft der Verriegelungsschraube und der Innenfläche der Querbohrung des Marknagels eine Verkeilung.

Das longitudinale Verkeilungselement kann ein Draht, vorzugsweise mit einem sich im Querschnitt verjüngenden Ende sein. Zweckmässigerweise besitzt dann der Durchgang der Verriegelungsschraube eine zum Drahtquerschnitt korrespondierende Bohrung.

Alternativ dazu kann das longitudinale Verkeilungselement ein Querschnittsprofil aufweisen, welches zum Querschnittsprofil des Durchgangs korrespondiert und vorzugsweise keilförmig ausgebildet ist.

Der Durchgang kann im wesentlichen kreisförmig ausgebildet sein mit einem Durchmesser von 0,5 - 2,0 mm, vorzugsweise von 0,8 - 1,2 mm.

Das longitudinale Verkeilungselement hat typischerweise eine Länge, welche mindestens zwei Drittel des Schraubenschaftes entspricht.

Das longitudinale Verkeilungselement kann an einem seiner Enden einen Anschlag für den Schraubenkopf aufweisen. Damit wird ein Durchstossen des Verkeilungselementes durch den Durchgang verhindert.

Der Durchgang kann auch mit einem Innengewinde versehen sein. Das longitudinale Verkeilungselement kann dann ebenfalls ein Aussengewinde tragen, welches mit dem Innengewinde des Durchgangs korrespondiert.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht einer Verriegelungsschraube mit einem Durchgang im Schraubenkopf und einem Verriegelungselement;
Fig. 2 einen Längsschnitt durch einen Marknagel in dessen Querbohrung die Verriegelungsschraube nach Fig. 1 eingesetzt ist;
Fig. 3 einen um 90° versetzten Längsschnitt durch den Marknagel nach Fig. 2 im Bereich seiner Querbohrung;
Fig. 4 eine perspektivische Ansicht einer modifizierten Verriegelungsschraube mit einer Bohrung im Schraubenkopf und einer Längsnut im Schraubenschaft;
Fig. 5 eine perspektivische Ansicht der Verriegelungsschraube nach Fig. 4 mit einem im Kopf der Schraube eingeführten longitudinalen Verkeilungselement;
Fig. 6 einen vergrösserten Längsschnitt durch die modifizierte Verriegelungsschraube nach Fig. 4;
Fig. 7 einen Querschnitt durch die modifizierte Verriegelungsschraube nach Fig. 4 im Bereich der Querbohrung des Marknagels;
Fig. 8 eine perspektivische Ansicht einer modifizierten Verriegelungsschraube mit einem schlüsselförmigen Durchgang und einem schlüsselförmigen Verriegelungselement;
Fig. 9 eine perspektivische Ansicht einer modifizierten Verriegelungsschraube mit zwei Nuten;
Fig. 10 einen Querschnitt durch die Verriegelungsschraube nach Fig. 9 in einer ersten, unverkeilten Position; und
Fig. 11 einen Querschnitt durch die Verriegelungsschraube nach Fig. 9 in einer zweiten, verkeilten Position; und
Fig. 12 eine schematische Querschnittdarstellung der Verriegelungsschraube nach Fig. 9 mit der ersten und zweiten Position der Verriegelungsschraube.

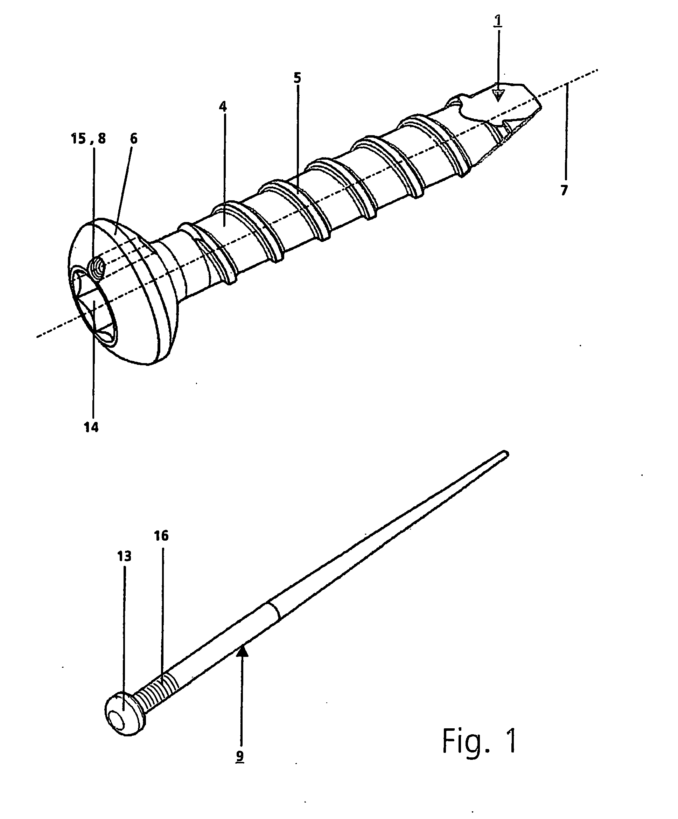

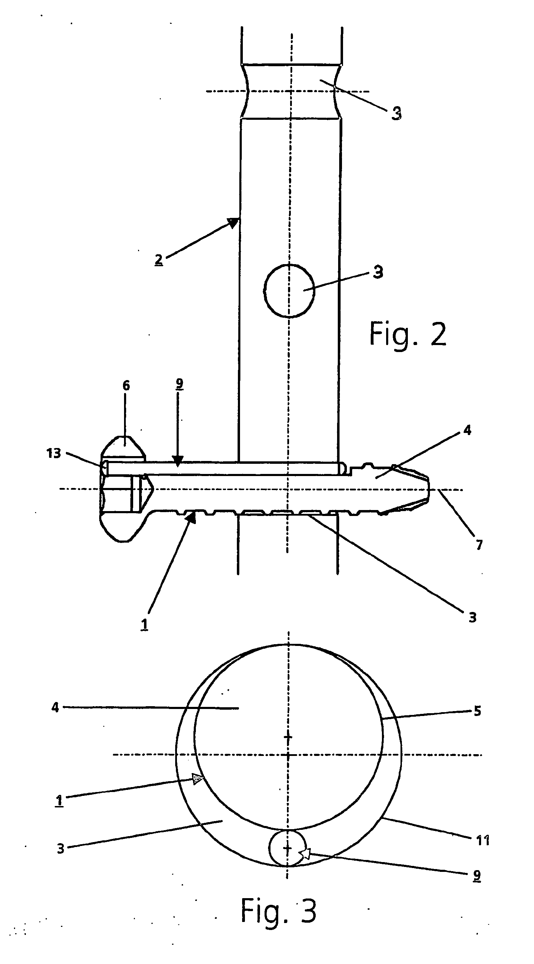

Die in Fig. 1 dargestellte Verriegelungsschraube 1 wird zur Verriegelung eines in Fig. 2 dargestellten, intramedullären Marknagels 2 verwendet, der über mehrere Querbohrungen 3 verfügt. Die Verriegelungsschraube 1 besitzt eine zentrale Längsachse 7 und umfasst einen Schraubenschaft 4, der mit einem Aussengewinde 5 versehen ist sowie einen Schraubenkopf 6 mit einem Innensechskant 14, um die Verriegelungsschraube 1 in eine der Querbohrungen 3 des Marknagels 2 eindrehen zu können.

Der Durchmesser des Schraubenkopfes 6 ist grösser ist als der Aussendurchmesser des Aussengewindes 5 wobei der Schraubenkopf 6 einen im wesentlichen parallel zur Längsachse 7 und zum Aussengewinde 5 verlaufenden Durchgang 8 in Form einer Kreisbohrung mit einem Innengewinde 15 zur Aufnahme eines longitudinalen Verkeilungselementes 9 in Form eines konisch zulaufenden Stiftes mit einem partiellen Aussengewinde 16 und einem kopfseitigen Anschlag 13, wobei das Aussengewinde 16 mit dem Innengewinde 15 korrespondiert. Das Verkeilungselement 9 hat eine Länge welche etwa zwei Drittel der Länge des Schraubenschaftes 4 entspricht.

In Fig. 3 ist dargestellt, wie das Verkeilungselement 9 zwischen der Innenfläche 11 der Querbohrung 3 und dem Aussendurchmesser des Aussengewindes 5 der Verriegelungsschraube 1 verkeilbar ist. Voraussetzung dazu ist der Umstand, dass der Aussendurchmesser des Aussengewindes 5 kleiner ist als der Durchmesser der Querbohrung 3.

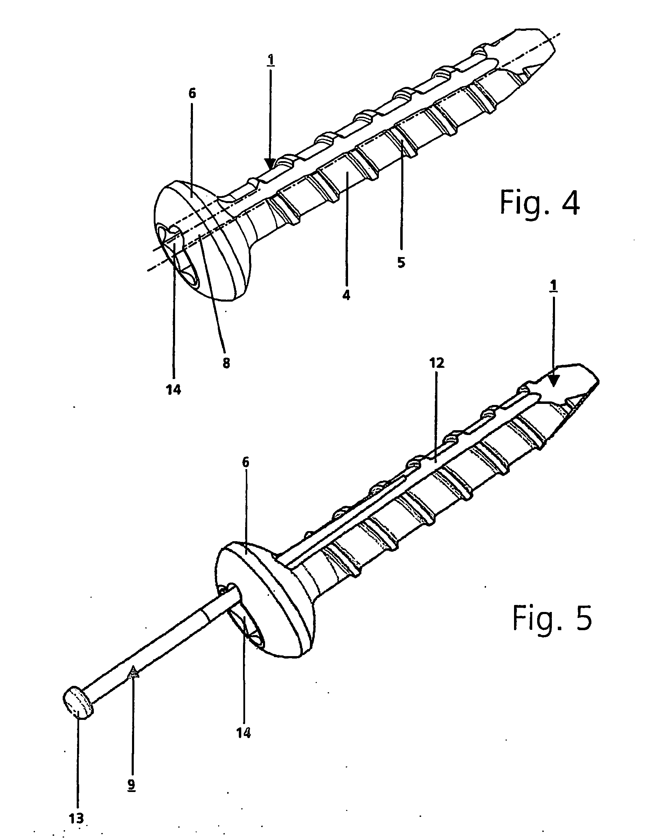

Wie in Fig. 2 und 5 dargestellt weist das longitudinale Verkeilungselement 9 an seinem hinteren Ende einen Anschlag 13 auf, so dass es nicht weiter in den Schraubenkopf 6 hineinstossbar ist. Dadurch wird verhindert, dass das Verkeilungselement 9 in den Markkanal gelangt.

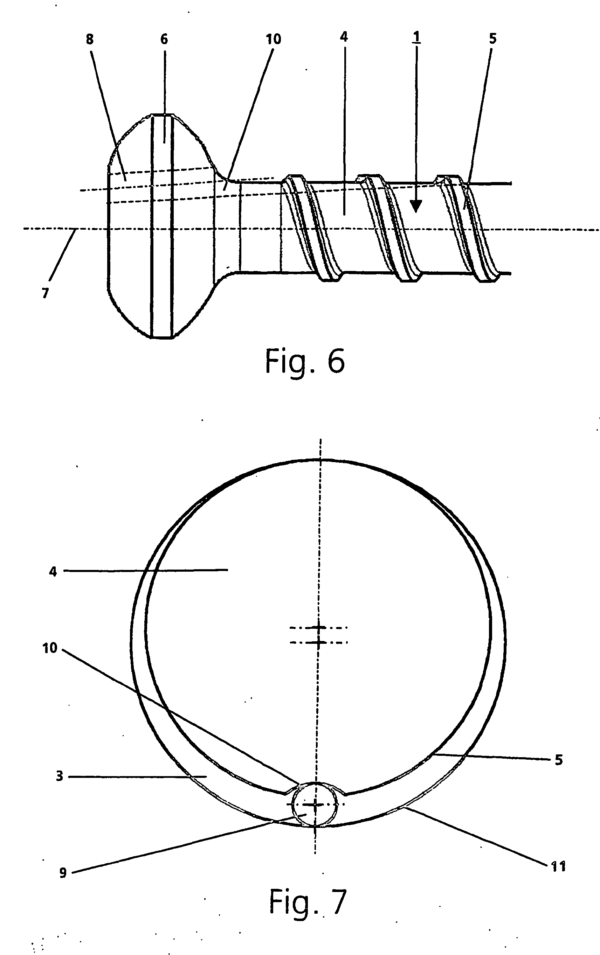

In den Fig. 4 - 7 ist eine Variante der Verriegelungsschraube 1 dargestellt, bei welcher der Schraubenschaft 4 eine mit dem Durchgang 8 (in Form einer Kreisbohrung mit einem Durchmesser von typischerweise 1 mm) fluchtende Nut 10 mit einem teilkreisförmigen Querschnitt aufweist. Die Nut 10 verjüngt sich konisch mit zunehmendem Abstand vom Schraubenkopf 6. Die Nut 10 weist eine Tiefe von 5 % Durchmessers des Schraubenschaftes 4 entspricht. Das ebenfalls konisch zulaufende Verkeilungselement 9 wird bei dieser Variante zwischen der Innenfläche 11 der Querbohrung 3 und der Innenfläche der Nut 10 verkeilt.

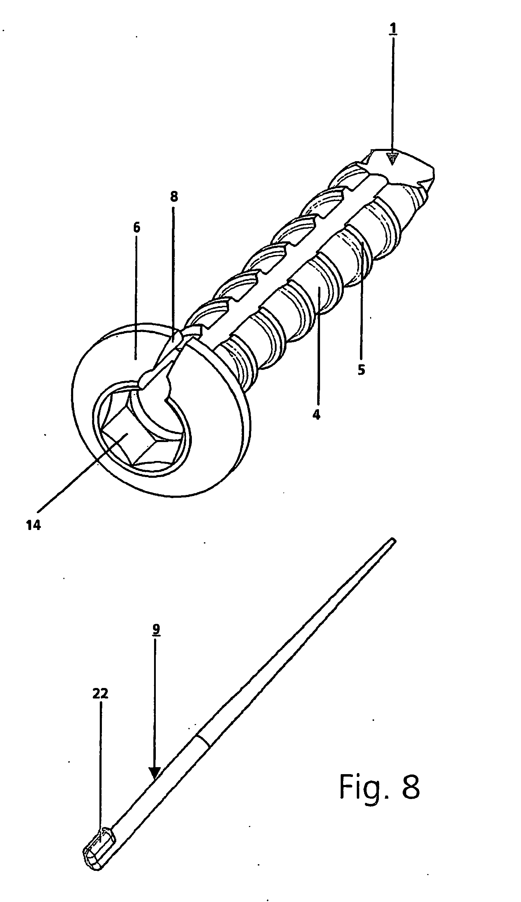

In Fig. 8 ist eine weitere Variante der Verriegelungsschraube 1 dargestellt, bei welcher der Durchgang 8 in Form einer schlüsselförmigen Nut realisiert ist zur Aufnahme eines longitudinalen Verkeilungselementes 9 in Form eines Stabes, welcher an seinem kopfseitigen Ende ein schlüsselförmiges Profil 22 aufweist, welches der schüsselförmigen Nut entspricht. Durch die Form des schlüsselförmigen Profils 22 wird das Verkeilungselement 9 gegen ein Verdrehen relativ zum Durchgang 8 gesichert.

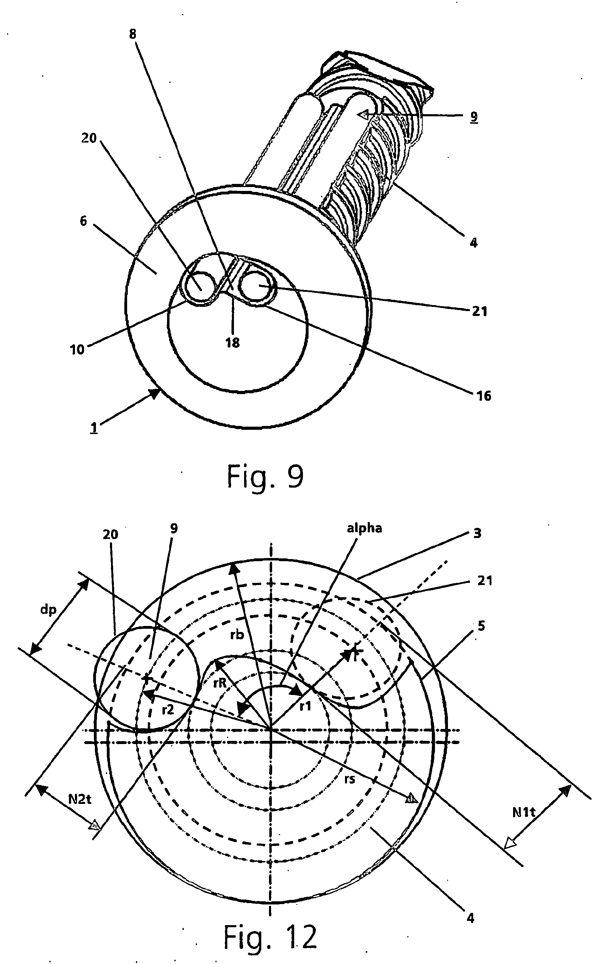

In den Fig. 9 bis 12 ist eine weitere Ausführungsform der Verriegelungsschraube 1 dargestellt. Der Schraubenschaft 4 weist bei dieser Variante neben einer ersten Nut 10 eine parallel zur ersten Nut 10 verlaufende, um den Rotationswinkel alpha von 50° radial versetzte, zweite Nut 16 auf, welche tiefer ist als die erste Nut 10. Dabei ist die erste Nut 10 über eine keilförmigen Rampe 18 mit der zweiten Nut 16 verbunden.
Der Durchgang 8 im Schraubenkopf 6 ist dabei als Langloch entsprechend der Geometrie der Rampe 18 ausgebildet, in welches beide Nuten 10,16 münden, so dass das longitudinale Verriegelungselement 9 sowohl in der ersten Nut 10 als auch in der zweiten Nut 16 plaziert werden kann.

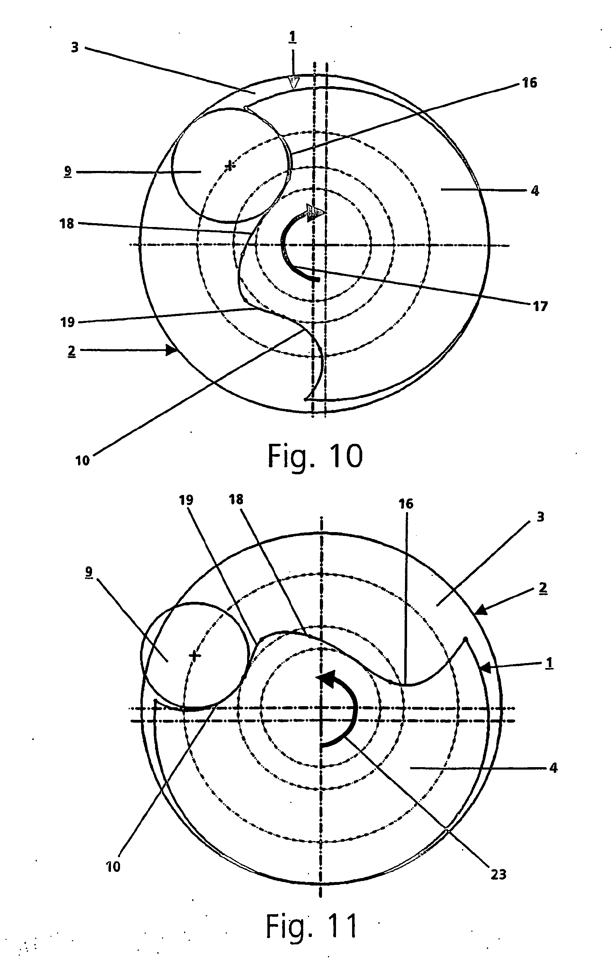

Durch Drehen der Verriegelungsschraube 1 in Richtung des Pfeiles 17 (Uhrzeigersinn) gleitet das in der zweiten Nut 16 liegende longitudinale Verriegelungselement 9 (unverkeilte Position in Fig. 10) entlang der relativ flachen, keilförmigen Rampe 18 bis es in die erste, weniger tiefe Nut 10 fällt (verkeilte Position in Fig. 11). Ein Zurückgleiten des longitudinalen Verriegelungselementes 9 von der ersten Nut 10 in die zweite Nut 16 wird durch die relativ steile Flanke 19 und die Überhöhung relativ zu den beiden Positionen 20 und 21 zwischen den beiden Nuten 10,16 verhindert. Das longitudinale Verriegelungselement 9 bewegt sich dabei im Langloch 8 von der Position 21 zur Position 20 (Fig. 9).

Durch Drehen der Verriegelungsschraube 1 in Richtung des Pfeiles 23 (Gegenuhrzeigersinn) fällt das in der ersten Nut 10 liegende longitudinale Verriegelungselement 9 (verkeilte Position in Fig. 11) wieder in die zweite Nut 16 (unverkeilte Position in Fig. 10), so dass die Verspannung der Verriegelungsschraube 1 wieder aufgehoben ist.

Wie in Fig. 12 dargestellt sollte die Dimensionierung der einzelnen Bauelemente vorteilhafterweise wie folgt sein:
- der Abstand zwischen dem Mittelpunkt des longitudinalen Verriegelungselementes 9 und dem Mittelpunkt des Schraubenschaftes 4 in Position 21 (gestrichelte Linien) beträgt r₁;
- der Abstand zwischen dem Mittelpunkt des longitudinalen Verriegelungselementes 9 und dem Mittelpunkt des Schraubenschaftes 4 in Position 20 (durchgehende Linien) beträgt r₂, wobei r₂ > r₁ ist;
- der Durchmesser des longitudinalen Verriegelungselementes 9 beträgt dₚ
- der Radius des Aussengewindes 5 des Schraubenschaftes 4 beträgt rₛ
- der Durchmesser der Querbohrung 3 beträgt r_{b} , wobei r_{b} ≧ rₛ
- die erwünschte Presspassung, resp. die Spielreduzierung zwischen dem Aussengewinde 5 des Schraubenschaftes 4, dem longitudinalen Verriegelungselementes 9 und der Querbohrung 3 des Marknagels ergibt sich, wenn die Summe von [(rₛ - N₂ₜ + dp)+ rₛ)] grösser oder gleich gross ist wie 2 r_{b}, wobei N₂ₜ die Nutentiefe in Position 20 bedeutet.

## Patentansprüche

1. Intramedullärer Marknagel (2) mit mindestens einer Querbohrung (3) und einer Verriegelungsschraube (1), welche in die Querbohrung (3) einführbar ist,
wobei
die Verriegelungsschraube (1) eine zentrale Längsachse (7) aufweist und folgende Elemente umfasst:
A) einen Schraubenschaft (4), der mindestens abschnittsweise mit einem Aussengewinde (5) versehen ist; und
B) einen Schraubenkopf (6), dessen Durchmesser grösser ist als der Aussendurchmesser des Aussengewindes (5), **dadurch gekennzeichnet, dass**
C) der Schraubenkopf (6) einen im Wesentlichen parallel zur Längsachse (7) und zum Aussengewinde (5) verlaufenden Durchgang (8) zur Aufnahme eines longitudinalen Verkeilungselementes (9) aufweist.

2. Intramedullärer Marknagel (2) nach Anspruch 1, **dadurch gekennzeichnet, dass** der Aussendurchmesser des Aussengewindes (5) der Verriegelungsschraube kleiner als der Durchmesser der Querbohrung (3) ist.

3. Intramedullärer Marknagel (2) nach Anspruch 1 oder 2, **dadurch gekennzeichnet, dass** er zusätzlich ein longitudinales Verkeilungselement (9) umfasst.

4. Intramedullärer Marknagel (2) nach Anspruch 3, **dadurch gekennzeichnet, dass** das longitudinales Verkeilungselement (9) in den Durchgang (8) der Verriegelungsschraube (1) einführbar ist, wobei das longitudinales Verkeilungselement (9) zwischen dem Schraubenschaft (4) der Verriegelungsschraube (1) und der Innenfläche (11) der Querbohrung (3) des Marknagels (2) verkeilbar ist.

5. Intramedullärer Marknagel (2) nach Anspruch 3 oder 4, **dadurch gekennzeichnet, dass** das longitudinale Verkeilungselement (9) ein Draht ist, vorzugsweise mit einem sich im Querschnitt verjüngenden Ende (12) und der Durchgang (8) der Verriegelungsschraube (1) eine zum Drahtquerschnitt vorzugsweise korrespondierende Bohrung ist.

6. Intramedullärer Marknagel (2) nach einem der Ansprüche 3 bis 5, **dadurch gekennzeichnet, dass** das longitudinale Verkeilungselement (9) ein Querschnittsprofil aufweist, welches zum Querschnittsprofil des Durchgangs (8) der Verriegelungsschraube (1) korrespondiert und vorzugsweise keilförmig ausgebildet ist.

7. Intramedullärer Marknagel (2) nach einem der Ansprüche 3 bis 6, **dadurch gekennzeichnet, dass** das longitudinale Verkeilungselement (9) eine Länge hat, welche mindestens zwei Drittel des Schraubenschaftes (4) entspricht.

8. Intramedullärer Marknagel (2) nach einem der Ansprüche 3 bis 7, **dadurch gekennzeichnet, dass** das longitudinale Verkeilungselement (9) an einem seiner Enden einen Anschlag (13) für den Schraubenkopf (6) aufweist.

9. Intramedullärer Marknagel (2) nach einem der Ansprüche 3 bis 8, **dadurch gekennzeichnet, dass** das, longitudinale Verkeilungselement (9) ein Aussengewinde (16) trägt, welches mit einem Innengewinde (15) des Durchgangs (8) korrespondiert.

10. Intramedullärer Marknagel (2) nach einem der Ansprüche 1 bis 9, **dadurch gekennzeichnet, dass** der Schraubenschaft (4) eine in Richtung des Durchgangs (8) verlaufende Nut (10), vorzugsweise mit einem teilkreisförmigen oder polygonalen Querschnitt, aufweist.

11. Intramedullärer Marknagel (2) nach Anspruch 10, **dadurch gekennzeichnet, dass** sich die Nut (10) mit zunehmendem Abstand vom Schraubenkopf (6) im Querschnitt verjüngt, vorzugsweise konisch.

12. Intramedullärer Marknagel (2) nach Anspruch 10 oder 11 **dadurch gekennzeichnet, dass** die Nut (10) einen Winkel zur Längsachse (7) aufweist, der vorzugsweise kleiner als 5° ist.

13. Intramedullärer Marknagel (2) nach einem der Ansprüche 1 bis 12, **dadurch gekennzeichnet, dass** der Durchgang (8) im Wesentlichen kreisförmig ausgebildet ist mit einem Durchmesser von 0,5 - 2,0 mm, vorzugsweise von 0,8 - 1,2 mm.

14. Intramedullärer Marknagel (2) nach einem der Ansprüche 10 bis 13, **dadurch gekennzeichnet, dass** die Nut (10) eine Tiefe aufweist, welche zwischen 1% und 50%, vorzugsweise 2 % bis 20 % des Durchmessers des Schraubenschaftes (4) entspricht.

15. Intramedullärer Marknagel (2) nach einem der Ansprüche 1 bis 14, **dadurch gekennzeichnet, dass** der Durchgang (8) mit einem Innengewinde (15) versehen ist.

## Claims

1. Intramedullary nail (2) with at least one transverse borehole (3) and a locking screw (1), which is insertable into the transverse borehole (3),
wherein
the locking screw (1) has a central longitudinal axis (7) and comprises the following elements:
A) a screw shaft (4) which is provided at least sectionally with an external thread (5); and
B) a screw head (6), the diameter of which is larger than the external diameter of the external thread (5),
**characterized in that**
C) the screw head (6) has a passage (8) for accommodating a longitudinal wedging element (9), the passage (8) extending essentially parallel to the longitudinal axis (7) and to the external thread (5).

2. Intramedullary nail (2) according to claim 1, **characterized in that** the external diameter of the external thread (5) is smaller than the diameter of the transverse borehole (3).

3. Intramedullary nail (2) according to claim 1 or 2, **characterized in that** it additionally comprises a longitudinal wedging element (9).

4. Intramedullary nail (2) according to claim 3, **characterized in that** the longitudinal wedging element (9) is introducible into the passage (8) of the locking screw (1), wherein the longitudinal wedging element (9) can be wedged between the screw shaft (4) of the locking screw (1) and the inner surface (11) of the transverse borehole (3) of the intramedullary nail (2).

5. Intramedullary nail (2) according to claim 3 or 4, **characterized in that** the longitudinal wedging element (9) is a wire, preferably with an end (12) tapering in cross-section and the passage (8) of the locking screw (1) is a borehole, preferably corresponding to the cross-section of the wire.

6. Intramedullary nail (2) according to one of the claims 3 to 5, **characterized in that** the longitudinal wedging element (9) has a cross-sectional profile, which corresponds to the cross-sectional profile of the passage (8) of the locking screw (1) and preferably is constructed wedge-shaped.

7. Intramedullary nail (2) according to one of the claims 3 to 6, **characterized in that** the longitudinal wedging element (9) has a length, which corresponds at least to two thirds of the screw shaft (4).

8. Intramedullary nail (2) according to one of the claims 3 to 7, **characterized in that** the longitudinal wedging element (9), at one of its ends, has a stop (13) for the screw head (6).

9. Intramedullary nail (2) according to one of the claims 3 to 8, **characterized in that** the longitudinal wedging element (9) carries an external thread (16), which corresponds to an internal thread (15) of the passage (8).

10. Intramedullary nail (2) according to one of the claims 1 to 9, **characterized in that** the screw shaft (4) has a groove (10) extending in the direction of the passage (8), preferably with a partially circular or polygonal cross-section.

11. Intramedullary nail (2) according to claim 10, **characterized in that** the groove (10) tapers in cross-section as its distance from the screw head (6) increases, preferably conically.

12. Intramedullary nail (2) according to claim 10 or 11, **characterized in that** the groove (10) encloses an angle with the longitudinal axis (7), which preferably is smaller than 5°.

13. Intramedullary nail (2) according to one of the claims 1 to 12, **characterized in that** the passage (8) is essentially circularly configured with a diameter of 0.5 - 2.0 mm and preferably of 0.8 - 1.2 mm.

14. Intramedullary nail (2) according to one of the claims 10 to 13, **characterized in that** the groove (10) has a depth, which corresponds to between 1% and 50% and preferably to 2% to 20% of the diameter of the screw shaft (4).

15. Intramedullary nail (2) according to one of the claims 1 to 14, **characterized in that** the passage (8) is provided with an internal thread (15).

## Revendications

1. Clou intramédullaire (2) comprenant au moins un perçage transversal (3) et une vis de blocage (1) qui peut être introduite dans le perçage transversal (3), où la vis de blocage (1) présente un axe longitudinal central (7) et comprend des éléments suivants :
A) une tige de vis (4) qui est dotée au moins partiellement d'un filetage extérieur (5) ; et
B) une tête de vis (6) dont le diamètre est supérieur au diamètre extérieur
du filetage extérieur (5),
**caractérisé en ce que**
C) la tête de vis (6) présente un passage (8) s'étendant sensiblement de façon parallèle à l'axe longitudinal (7) et au filetage extérieur (5) et servant à recevoir un élément de clavetage longitudinal (9).

2. Clou intramédullaire (2) selon la revendication 1, **caractérisé en ce que** le diamètre extérieur du filetage extérieur (5) de la vis de blocage est inférieur au diamètre du perçage transversal (3).

3. Clou intramédullaire (2) selon la revendication 1 ou 2, **caractérisé en ce qu'**il comprend en outre un élément de clavetage longitudinal (9).

4. Clou intramédullaire (2) selon la revendication 3, **caractérisé en ce que** l'élément de clavetage longitudinal (9) peut être introduit dans le passage (8) de la vis de blocage (1), où l'élément de clavetage longitudinal (9) peut être claveté entre la tige de vis (4) de la vis de blocage (1), et la surface intérieure (11) du perçage transversal (3) du clou intramédullaire (2).

5. Clou intramédullaire (2) selon la revendication 3 ou 4, **caractérisé en ce que** l'élément de clavetage longitudinal (9) est un fil, de préférence comprenant une extrémité (12) diminuant en section, et le passage (8) de la vis de blocage (1) est un perçage correspondant de préférence à la section du fil.

6. Clou intramédullaire (2) selon l'une quelconque des revendications 3 à 5, **caractérisé en ce que** l'élément de clavetage longitudinal (9) présente un profil de section qui correspond au profil de section du passage (8) de la vis de blocage (1) et est configuré de préférence en forme de clavette.

7. Clou intramédullaire (2) selon l'une quelconque des revendications 3 à 6, **caractérisé en ce que** l'élément de clavetage longitudinal (9) a une longueur qui correspond au moins aux deux tiers de la tige de vis (4).

8. Clou intramédullaire (2) selon l'une quelconque des revendications 3 à 7, **caractérisé en ce que** l'élément de clavetage longitudinal (9) présente, au niveau de l'une de ses extrémités, une butée (13) pour la tête de vis (6).

9. Clou intramédullaire (2) selon l'une quelconque des revendications 3 à 8, **caractérisé en ce que** l'élément de clavetage longitudinal (9) comprend un filetage extérieur (16) qui correspond à un filetage intérieur (15) du passage (8).

10. Clou intramédullaire (2) selon l'une quelconque des revendications 1 à 9, **caractérisé en ce que** la tige de vis (4) présente une rainure (10) s'étendant en direction du passage (8) et ayant de préférence une section de forme partiellement circulaire ou polygonale.

11. Clou intramédullaire (2) selon la revendication 10, **caractérisé en ce que** la rainure (10) diminue en section, de préférence de façon conique, en ayant un intervalle croissant par rapport à la tête de vis (6).

12. Clou intramédullaire (2) selon la revendication 10 ou 11, **caractérisé en ce que** la rainure (10) présente un angle par rapport à l'axe longitudinal (7), angle qui est de préférence inférieur à 5°.

13. Clou intramédullaire (2) selon l'une quelconque des revendications 1 à 12, **caractérisé en ce que** le passage (8) est configuré sensiblement de forme circulaire, avec un diamètre de 0,5 mm à 2,0 mm, de préférence de 0,8 mm à 1,2 mm.

14. Clou intramédullaire (2) selon l'une quelconque des revendications 10 à 13, **caractérisé en ce que** la rainure (10) présente une profondeur qui correspond à des valeurs comprises entre 1 % et 50 %, de préférence entre 2 % et 20 % du diamètre de la tige de vis (4).

15. Clou intramédullaire (2) selon l'une quelconque des revendications 1 à 14, **caractérisé en ce que** le passage (8) est doté d'un filetage intérieur (15).
